# EUROPEAN PATENT APPLICATION

(11) **EP 4 299 035 A1**
(43) Date of publication of application: **03.01.2024**
(21) Application number: 22874473.6
(22) Date of filing: 19.08.2022
(51) Int. Cl.: A61F 2/24

(54) **DELIVERY CATHETER, IMPLANT DELIVERY SYSTEM, AND WORKING METHOD**

(30) Priority: 28.09.2021 CN 202111145941
(71) Applicant: Jiangsu Trulive Medtech Co., Ltd, Rudong County Nantong Jiangsu 226400 (CN)
(72) Inventor: SUN, Yunyan, Nantong, Jiangsu 226400 (CN); ZHAO, Jing, Nantong, Jiangsu 226400 (CN); WEN, Jing, Nantong, Jiangsu 226400 (CN)
(74) Representative: Nordmeyer, Philipp Werner
(86) International application number: PCT/CN2022/113498
(87) International publication number: WO 2023/051078

(57) **Abstract**

A delivery catheter (1), an implant delivery system and methods of operation. A balloon structure in the delivery catheter (1) includes an expandable component and a balloon body (140). The expandable component is disposed within the balloon body (140) so as to be expandable therein. Moreover, the expandable component is sleeved over a distal end of a guidewire tube (210). An implant is disposed over the balloon body (140) and axially restrained over a middle portion of the expandable component during delivery. The expandable component is configured to provide a radially outward support force from a middle portion of the balloon body (140) during expansion so as to bring internal environments of the balloon body (140) respectively at distal and proximal end thereof into communication. This facilitates stable and uniform and hence symmetric expansion of the implant. Moreover, it is helpful in addressing the risk of sudden axial movement of the implant with respect to a delivery system during delivery and release.

## Description

### TECHNICAL FIELD

The present invention relates to the field of medical devices, and in particular to a delivery catheter, an implant delivery system and methods of operation.

### BACKGROUND

Human heart valves include the aortic valve, the pulmonary valve, the mitral valve and the tricuspid valve. All these heart valves function like one-way valves to maintain continual blood flow in the human body in a specific direction, thereby establishing effective blood circulation. However, these heart valves may fail to function due to congenital defects, inflammation, degenerative aging or other reasons, leading to circulatory disorders which may cause severe damage to cardiac blood vessels and organs, or even death. In the current clinical practice, surgical repair or replacement provide the most effective treatment to defective native valves. However, conventional surgical valvuloplasty is much invasive, requires a long time for a patient to recover and tends to be associated with multiple complications. In contrast, interventional valvuloplasty can implant a valve prosthesis or an artificial heart valve at the location of a defective native valve to achieve a therapeutic effect in a less invasive way by delivering it through a large blood vessel in the patient's body.

Transcatheter aortic valve replacement (TAVR) is catheterized intervention procedure involving first crimping an artificial valve into a delivery system outside the patient's body and then delivering the artificial valve to the annulus of the patient's native aortic valve through a vascular or trans-apical access, followed by release of it at the location of the annulus in order to replace the native valve. At present, common artificial valves for use in TAVR applications are mainly self-expanding valves and balloon-expandable valves. Both these types of artificial valves are crimped into a delivery system before being delivery into a human body. During release, a self-expanding valve spontaneously expands to replace a defective native valve by virtue of temperature-dependent phase transitions of a specialized metal such as a nickel-titanium alloy from which a stent in the valve is fabricated. In contrast, a balloon-expandable valve is unfolded to replace a defective native valve by external forces exerted by through a balloon or another expansion tool.

Compared with self-expanding valves, balloon-expandable valves typically exhibit higher radial support and better roundness after release. However, since valve release must resort to external forces, the requirements on the delivery system used to achieve the release are more demanding so as to achieve a good match between the delivery system and prosthetic valve. Despite the rapid development of valve replacement techniques using balloon-expandable valves, there are still some challenges in the design of associated delivery systems. For example, it is difficult to ensure accurate and stable delivery and release of an artificial valve. With the existing designs, during the delivery and release of an artificial valve using a delivery system, there remains a risk of sudden axial movement of valve with respect to the delivery system, which, in severe cases, can possibly lead to incomplete or asymmetric unfolding of the artificial valve, or even damage to the delivery system and dislodgement of the valve.

### SUMMARY OF THE INVENTION

It is an object of the present invention to provide a delivery catheter, an implant delivery system and methods of operation, which facilitate the resolution of the problem of a risk of sudden axial movement of an implant with respect to a delivery system and risks associated with release of the implant during delivery and release of the implant by the delivery system.

It is another object of the present invention to overcome the problem of a risk of sudden axial movement of an implant with respect to a delivery system and risks associated with release of the implant during delivery and release of the implant by the delivery system.

To this end, the present invention provides a delivery catheter comprising a catheter, a balloon structure and a guidewire tube, the balloon structure disposed at a distal end of the catheter, the guidewire tube inserted within the catheter and the balloon structure,
the balloon structure comprising an expandable component and a balloon body, the expandable component disposed within the balloon body so as to be expandable therein, the expandable component disposed at a distal end of the guidewire tube, with an implant being sleeved over the balloon body and being axially restrained over a middle portion of the expandable component during delivery, the expandable component configured to provide a radially outward support force from a middle portion of the balloon body during expansion so as to bring internal environments of the balloon body respectively at distal and proximal end thereof into communication.

Optionally, the implant may comprise an artificial valve.

Additionally, the balloon structure may further comprise a proximal support member and a distal support member, which are both provided within the balloon body, wherein each of the proximal and the distal support members has a same axial direction as the expandable component, the proximal support member sleeved over a proximal end of the expandable component, the distal support member sleeved over a distal end of the expandable component, wherein the artificial valve is axially restrained between the proximal and distal support members during delivery.

Additionally, the expandable component may be an inflatable balloon body comprising a first section, a second section and a third section, that are sequentially connected from its proximal to distal end, the first section inserted within the proximal support member and proximally connected to an inner wall of the catheter, the third section inserted within the distal support member and distally connected to an outer wall of the guidewire tube, the second section located between the proximal and distal support members, wherein the artificial valve is arranged over the second section during delivery.

Additionally, each of the first section and the third section may have at least one indentation stripe arranged circumferentially, wherein the at least one indentation stripe is provided on an outer wall of the inflatable balloon body, and wherein an extension direction of the indentation stripe is as same as an axial direction of the inflatable balloon body.

Additionally, the indentation stripe may comprise at least one indentation spaced axially.

Additionally, the expandable component may be a secondary balloon, a distal end of the secondary balloon is inserted within the distal support member and is connected to the outer wall of the guidewire tube, and wherein a proximal end of the secondary balloon is inserted within the proximal support member and communicates with the catheter.

Additionally, the proximal support member may be a tubular structure open at both ends, a tapered resilient member or a multi-ridge clamp, and the distal support member may be a tubular structure, a resilient member or a multi-ridge clamp.

Additionally, in case of the proximal support member and/or the distal support member being tubular structure(s), a plurality of through-pores may be even distributed on an outer circumferential surface of the tubular structure(s).

In case of the proximal support member and/or the distal support member being tapered resilient member(s), tapered resilient member(s) may comprise a maximum diameter at its end proximal to the artificial valve.

In a second aspect, the present invention provides an implant delivery system comprising the delivery catheter as defined above.

In a third aspect, the present invention provides a method for operating the implant delivery system, in which the expandable component is implemented as the inflatable balloon body as defined above. The method includes:
crimping the implant onto the balloon structure, wherein the balloon body fits against the inflatable balloon, and wherein the implant is axially restrained between the proximal and distal support members;
delivering the implant to a predetermined site;
introducing an inflation fluid from a proximal end of the catheter, wherein the inflation fluid then flows through the catheter into the inflatable balloon, wherein the implant initially expands along an radial direction through the inflatable balloon, and wherein the communication between the internal environments of the balloon body at the distal and proximal ends thereof is established;
increasingly pressurizing the inflation fluid to cause tearing of the indentation stripes under the action of the pressure, bringing the interior of the inflatable balloon into communication with the exterior thereof, wherein the inflation fluid flows at the same rate and the same pressure into the balloon body through the first and third sections;
inflating the balloon body to its maximum possible size so as to fully expand the implant; and
releasing the implant to the predetermined site.

In a fourth aspect, the present invention provides a method for operating the implant delivery system, in which the expandable component is implemented as the secondary balloon as defined above. The method includes:
crimping the implant onto the balloon structure, wherein the balloon body fits against the secondary balloon, and wherein the implant is axially restrained between the proximal and distal support members;
delivering the implant to a predetermined site;
introducing an inflation fluid from a proximal end of the catheter, wherein the inflation fluid then flows through the catheter into the secondary balloon, wherein the implant initially expands along a radial direction through the secondary balloon, and wherein the communication between the internal environments of the balloon body at the distal and proximal ends thereof is established;
deflating the secondary balloon;
introducing the inflation fluid through the catheter into the balloon body, and inflating the balloon body to its maximum size so as to fully expand the implant; and
releasing the implant to the predetermined site.

Compared with the prior art, the present invention has at least the following benefits:
1. The expandable component provides a radially outward support force from the middle portion of the balloon body during expansion so as to bring the internal environments of the balloon body respectively at the distal and proximal end thereof into communication. This facilitates stable and uniform and hence symmetric expansion of the implant.
2. The implant is axially restrained between the proximal and distal support members and thus prevented from sudden axial movement with respect to a delivery and release once it is crimped onto the expandable component (e.g., the inflatable balloon, or the secondary balloon).
3. The indentations provided in the inflatable balloon on the opposite ends of the implant enable the internal environments of the balloon body respectively at the proximal and distal ends thereof to be brought into communication with each other during release of the implant. In this way, the balloon body can be inflated simultaneously at the proximal and distal ends, avoiding inadvertent dislodgement of the implant due to inflation of the balloon body non-simultaneously at the proximal and distal ends thereof. When the indentations in the expandable component are torn at a high pressure, the inflation fluid will flow into the balloon body through the indentations at the same rate and the same pressure, thereby inflating the balloon body simultaneously at the proximal and distal thereof. This enables stable and uniform and hence symmetric expansion of the implant.

### BRIEF DESCRIPTION OF THE DRAWINGS

Figs. 1a to 1c are schematic diagrams illustrating the structure of a delivery catheter according to a first embodiment of the present invention.
Fig. 2a schematically illustrates a configuration of proximal and distal support members according to the first embodiment of the present invention.
Fig. 2b schematically illustrates another configuration of the proximal and distal support members according to the first embodiment of the present invention.
Figs. 3a to 3c are schematic diagrams structurally showing an inflatable balloon according to the first embodiment of the present invention which is undergoing an inflation process.
Fig. 4 is a schematic diagram showing the structure of a delivery catheter according to a second embodiment of the present invention.
Fig. 5 is a schematic diagram showing the structure of a balloon body of a secondary balloon according to the second embodiment of the present invention.

### In these figures,

1, a delivery catheter; 10, an artificial valve; 110, a proximal support member; 120, a distal structure; 121, a distal support member; 122, a connecting portion; 123, a tapered tip; 130, an inflatable balloon; 131, a first section; 132, a second section; 133, a third section; 134, an indentation stripe; 1341, an indentation; 140, a balloon body;
210, a guidewire tube; 220, a catheter; and
300, a secondary balloon.

### DETAILED DESCRIPTION

Delivery catheters, implant delivery systems and methods of operation proposed herein will be described in greater detail below. The present invention will be described in greater detail below with reference to the accompanying drawings, which present preferred embodiments of the invention. It would be appreciated that those skilled in the art can make changes to the invention disclosed herein while still obtaining the beneficial results thereof. Therefore, the following description shall be construed as being intended to be widely known by those skilled in the art rather than as limiting the invention.

For the sake of clarity, not all features of actual implementations are described. In the following, description and details of well-known functions and structures are omitted to avoid unnecessarily obscuring the invention. It should be appreciated that in the development of any such actual implementation, numerous implementation-specific decisions must be made to achieve specific goals of the developers, such as compliance with system-related and business-related constrains, which may vary from one implementation to another. Moreover, it should be appreciated that such a development effort might be complex and time consuming, but would nevertheless be a routine undertaking for those of ordinary skill in the art.

Objects and features of the present invention will become more apparent upon reading the following more detailed description thereof made with reference to the accompanying drawings and to particular embodiments. Note that the figures are provided in a very simplified form not necessarily drawn to exact scale and for the only purpose of facilitating easy and clear description of the disclosed embodiments. As used herein, the term "or" is generally employed in the sense of "and/or", unless the context clearly dictates otherwise. As used herein, the terms "inner", "outer" and similar terms are merely illustrative and do not represent the only implementation possible. As used herein, the terms "proximal end" and "distal end" are employed to describe relative orientations, relative positions and directions between components of a medical device or actions thereof, as viewed by a physician operating the device. Without wishing to be limiting, a "proximal end" usually refers to an end closer to the operator, and a "distal end" usually refers to an end closer to the heart of a patient, during normal operation of the medical device.

In principle, the present invention seeks to provide a delivery catheter, an implant delivery system and methods of operation. The delivery catheter includes a catheter, a balloon structure and a guidewire tube. The balloon structure is provided at a distal end of the catheter, and the guidewire tube is inserted within the catheter and the balloon structure.

The balloon structure includes an expandable component and a balloon body. The expandable component is disposed within the balloon body and is sleeved over a distal end of the guidewire tube. An implant is arranged on the balloon body and is axially restrained over a middle portion of the expandable component during a delivery process. The expandable component is configured to provide a radially outward support force from the middle portion of the balloon body and thereby bring internal environments of the balloon body respectively at its distal and proximal ends into communication with each other during an expansion process.

When the delivery catheter is an inflatable balloon, a method for use with the implant delivery system includes:
crimping the implant onto the balloon structure so that the balloon body fits against the inflatable balloon, and the implant is axially restrained between the proximal and distal support members;
delivering the implant to a predetermined site;
introducing an inflation fluid from a proximal end of the catheter, which then flows through the catheter into the inflatable balloon, wherein the implant initially expands along a radial direction by the inflatable balloon and a communication is established between the internal environments of the balloon body respectively at its distal and proximal ends;
increasingly pressurizing the inflation fluid to cause tearing of indentation stripes under the action of the pressure, bringing the interior of the inflatable balloon into communication with the exterior of the inflatable balloon, and the inflation fluid flows at the same rate and the same pressure into the balloon body through the first and third sections;
inflating the balloon body to its maximum size so as to fully expand the implant; and
releasing the implant to the predetermined site.

When the delivery catheter is a secondary balloon, a method for use with the implant delivery system includes:
crimping the implant onto the balloon structure so that the balloon body fits against the secondary balloon, and the implant is axially restrained between the proximal and distal support members;
delivering the implant to a predetermined site;
introducing an inflation fluid from a proximal end of the catheter, which then flows through the catheter into the secondary balloon, wherein the implant initially expands along a radial direction by the secondary balloon and a communication is established between the internal environments of the balloon body respectively at its distal and proximal ends;
deflating the secondary balloon;
introducing the inflation fluid through the catheter into the balloon body, and inflating the balloon body to its maximum size so as to fully expand the implant; and
releasing the implant to the predetermined site.

### Embodiment 1

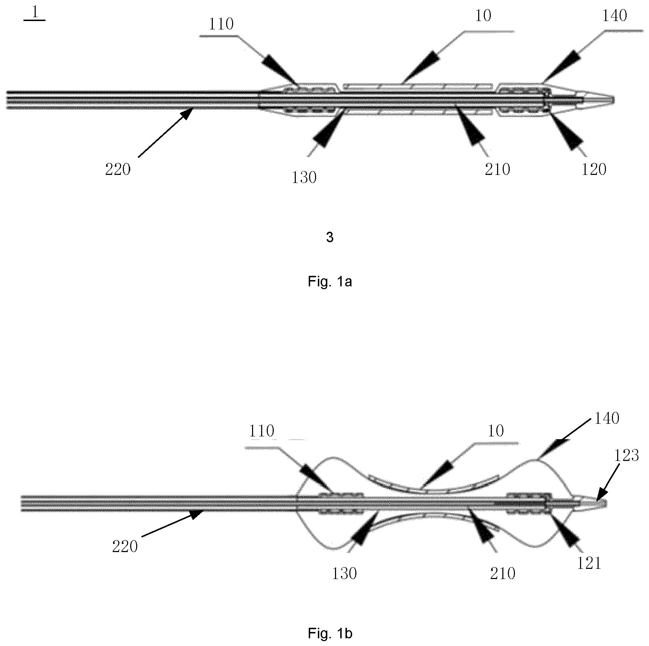

As shown in Figs. 1a to 1c, in a first embodiment, there is provided an implant delivery system including a delivery catheter. The delivery system is used to delivery an implant including an artificial valve. A particular example of the artificial valve is a balloon-expandable valve.

The delivery catheter includes a catheter, a guidewire tube and a balloon structure. The guidewire tube is inserted within and through the catheter, and a distal end of the guidewire tube protrudes out of a distal end of the catheter. A diameter of the guidewire tube is smaller than a diameter of the catheter, allowing an inflation fluid to flow into the catheter from a proximal end thereof over the guidewire tube and out of the catheter from the distal end of the catheter. The balloon structure is configured for loading and delivery of the artificial valve and provided at the distal end of the catheter. The balloon structure is sleeved over a distal end of the guidewire tube.

The balloon structure includes a proximal support member, a distal structure, an inflatable balloon and a balloon body. The proximal support member is disposed at the proximal end of the catheter, and the distal structure is provided at the distal end of the guidewire tube. The inflatable balloon is sleeved over the guidewire tube and is proximally inserted within the proximal support member. Moreover, the inflatable balloon is distally inserted within the distal structure. The balloon body is sleeved over the proximal support member, the distal structure and the inflatable balloon, and is proximally fixed to an outer wall of the catheter. Further, the balloon body is distally fixed to an outer wall of the distal structure.

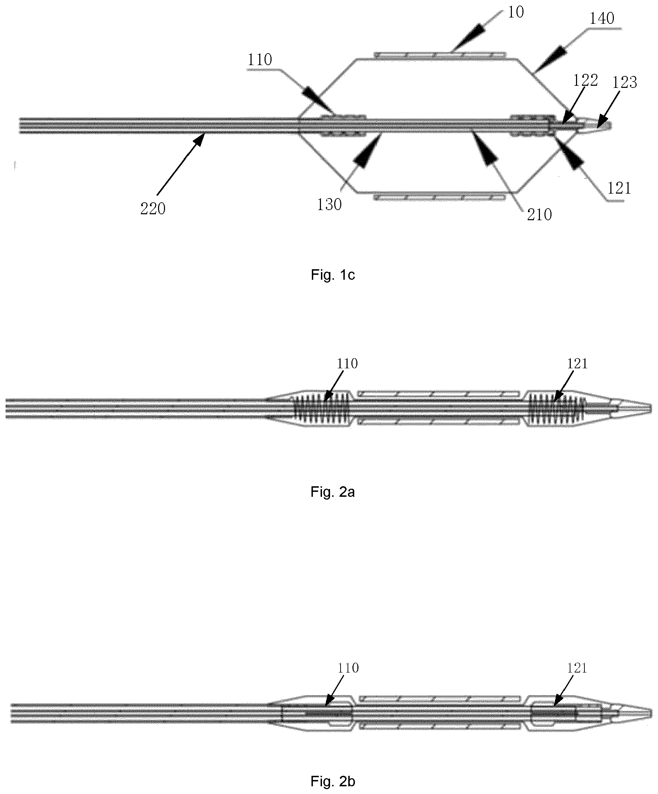

The proximal support member is fixedly connected to the proximal end of the catheter by welding, adhesive bonding or integral injection molding. The proximal support member may be a tubular structure open at both ends. Preferably, multiple through-pores are provided in a circumferential surface of the tubular structure (i.e., a multi-pore tubular structure, as particularly shown in Figs. 1a to 1c), which extend through the tubular structure radially. These impart increased flexibility to the proximal support member, facilitating crimping of the proximal support member into the balloon body during fabrication. Alternatively, the proximal support member may be a tapered resilient member (e.g., a tapered spring, as particularly shown in Fig. 2a), a multi-ridge clamp (as shown in Fig. 2b) or the like. The multi-ridge clamp is a tubular structure open at both ends and with multiple circumferentially spaced grooves in its end portion. The grooves extend axially to divide an end portion of the tubular structure into multiple leaf-like parts which are the clamping ridges of the multi-ridge clamp. The multi-ridge clamp may have three such clamping ridges, for example.

The distal structure is sleeved over the distal end of the guidewire tube and includes, axially connected together in sequence from its proximal to distal end, a distal support member, a connecting portion and a tapered tip. The tapered tip has a diameter gradually decreasing from its proximal to distal end, which facilitates introduction of the delivery catheter to a blood vessel. Preferably, the balloon body is distally fixed to an outer wall of the tapered tip around the proximal end of the tapered tip. The tapered tip comprises a lumen extending axially therethrough. Both the distal support member and the connecting portion may be tubular structures open at both ends. An outer diameter of the connecting portion may be smaller than an outer diameter of the distal support member, and an inner diameter of the connecting portion may be smaller than an inner diameter of the distal support member and may be slightly greater than an outer diameter of the guidewire tube. An outer surface of the guidewire tube may be adhesively bonded to an inner surface of the connecting portion and terminate at a distal end of the connecting portion. The guidewire tube may be coaxial with the lumen to allowing smooth passage of a guidewire therethrough.

Preferably, multiple through-pores are provided in a circumferential surface of the distal support member (i.e., a multi-pore tubular structure, as particularly shown in Figs. 1a to 1c), which extend through the tubular structure radially. These impart increased flexibility to the distal support member, facilitating crimping of the distal structure into the balloon body during fabrication. Alternatively, the distal support member may be a tapered resilient member (e.g., a tapered spring, as particularly shown in Fig. 2a), a multi-ridge clamp (as shown in Fig. 2b) or the like. The multi-ridge clamp is a tubular structure, and a distal portion of the clamp is divided into three clamping ridges, three clamping ridges are radially moveable to vary a diameter at the distal end. Both the proximal and distal support members may be multi-pore tubular structures, tapered springs or multi-ridge clamps. Alternatively, the proximal and distal support members may be different ones of a multi-pore tubular structure, a tapered spring and a multi-ridge clamp.

The inflatable balloon is distally inserted within the distal structure and fixedly connected by welding, bonding or otherwise to an outer wall of the guidewire tube at the distal end thereof so that the inflatable balloon is closed at a distal end of the balloon structure. The inflatable balloon may be proximally inserted within the catheter and fixedly connected by welding, bonding, anchoring or otherwise to an inner wall of the catheter around the distal end thereof, allowing an inflation fluid introduced into the catheter to enter the inflatable balloon from the proximal end of the inflatable balloon and flow distally.

The inflatable balloon may have an elongate shape, particularly such as a cylindrical, lageniform or cuboid shape. The inflatable balloon may have the same axial direction as the guidewire tube. Preferably, the inflatable balloon is cylindrical. The inflatable balloon may be made of a polymeric material, such as PA, PE, PP, PEBAX or silicone rubber. Preferably, the inflatable balloon is made of PA.

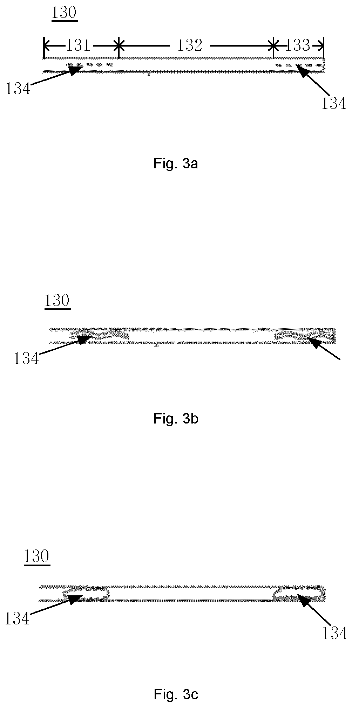

As shown in Figs. 3a to 3c, the inflatable balloon includes, axially connected together in sequence from its proximal to distal end, a first section, a second section and a third section. The second section is located between the proximal and distal support members. The first section is inserted within the proximal support member, and the third section is inserted within the distal structure. Referring to Fig. 1a, in a collapsed configuration (prior to the introduction of any inflation fluid), the artificial valve fits over the proximal support member, the distal structure and the second section, thereby maintaining the surface shape of the proximal support member, the distal structure and the second section. The artificial valve is sleeved over the balloon body across the second section. As both the proximal and distal support members have a diameter greater than a diameter of the inflatable balloon, the crimped artificial valve can be firmly embedded over the second section between the proximal and distal support members. In this way, the artificial valve is axially restrained between the proximal and distal support members without sudden axial movement during delivery and release.

Each of the first and third sections of the inflatable balloon has at least one indentation stripe on an outer wall thereof. Preferably, there are 2-6 indentation stripes on the outer wall of each of the first and third sections of the inflatable balloon. All the indentation stripes are even spaced apart circumferentially around the first and third sections and extend in the same axial direction as that of the inflatable balloon.

As shown in Fig. 3a, each indentation stripe includes at least one indentation. In case of multiple indentations in each indentation stripe, all these indentations are spaced apart at intervals, which are small enough to allow adjacent indentations in each indentation stripe to be brought into communication during an inflation process, as shown in Fig. 3b. The indentations have a smaller thickness than remaining portion of the inflatable balloon such that the inflatable balloon can be easily torn when impacted by a highly-pressurized inflation fluid, thus bringing the interior of the inflatable balloon into the exterior thereof. As such, after the artificial valve is released, microstructures can be formed to slightly expand the valve, bringing internal environments at the proximal and distal ends of the balloon body into communication with each other. This enables the balloon body to be inflated simultaneously at both its proximal and distal ends, avoiding dislodgement of the artificial valve that may result from inflation of the balloon body non-simultaneously at the proximal and distal end, as shown in Fig. 3c. The indentations may be formed, for example, by laser etching, chemical etching or physical etching. The indentations may have a circular, square or other regular shape.

During use, at first, as shown in Fig. 1a, the artificial valve is crimped onto the balloon body by an external force. At this stage, the balloon body has a collapsed configuration without any inflation fluid in the inflatable balloon (i.e., a non-inflated configuration). In this configuration, the artificial valve is fully crimped and fit against the outer side of the balloon structure and be axially restrained between the proximal support member and the distal structure. Next, as shown in Figs. 1b and 3b, an inflation fluid is introduced to the catheter from the proximal end thereof at a relatively low pressure and then flows through the catheter into the inflatable balloon from its proximal end, overall inflating the inflatable balloon. At this time, because of the flexibility of the inflatable balloon, the inflatable balloon located between the proximal support member and the distal structure can slightly expand the artificial valve outwardly, resulting in the formation of microstructures. As a result of the outward expansion of the inflatable balloon, adjacent indentations in each indentation stripe on the outer wall of the inflatable balloon are brought into communication, and the internal environments at the proximal and distal ends of the balloon body are also brought into communication with each other. This greatly reduces the risk of dislodgement of the artificial valve that may result from inflation of the balloon body successively at its proximal and distal ends. Subsequently, as shown in Figs. 1c and 3c, the inflation fluid is increasingly pressurized to cause tearing of the indentations in the indentation stripes. As a result, the inflation fluid flows from the inflatable balloon into the balloon body through the indentations in the indentation stripes. Since the indentation stripes are formed in the outer wall of the inflatable balloon in both the proximal first section and the distal third section, the inflation fluid flows into the balloon body through the openings formed in the proximal and distal sections of the inflatable balloon at the same rate and the same pressure until the balloon body is uniformly inflated to its maximum size. This enables stable release of the artificial valve and solves the problem of incomplete or asymmetric expansion of the valve during release.

### Embodiment 2

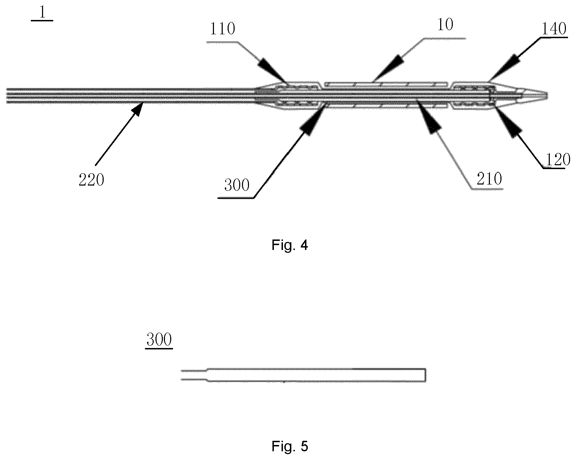

In a second embodiment, a secondary balloon is used in place of the inflatable balloon in the first embodiment. Fig. 4 is a schematic diagram showing the structure of a delivery catheter according to the second embodiment. Fig. 5 is a schematic diagram showing the structure of a main body of the secondary balloon according to the second embodiment. As shown in Figs. 4 and 5, the secondary balloon is sleeved over a guidewire tube and distally closed against an outer wall of the guidewire tube around a distal end of the guidewire. Moreover, the secondary balloon is distally inserted within a distal structure and proximally communicates with a catheter. A balloon body is sleeved over the secondary balloon, a proximal support member and the distal structure and proximally communicates with the catheter. The secondary balloon and the balloon body may respectively communicate with the same catheter, and a valve may be provided to enable an inflation fluid to flow into the secondary balloon and the balloon body at different times. Alternatively, the secondary balloon and the balloon body may communicate with different catheters. In this way, the secondary balloon and the balloon body can be inflated and deflated independently of each other. Of course, the secondary balloon and balloon body may be brought into communication with the catheters using any suitable method known in the art, as long as the secondary balloon and the balloon body can be inflated and deflated independently of each other.

During use, an artificial valve is crimped onto the balloon body by an external force. At this stage, the balloon body is brought into a collapsed configuration without any inflation fluid in the secondary balloon. In this configuration, the artificial valve is fully crimped and fit against the outer side of the balloon structure and be axially restrained between the proximal support member and the distal structure. Next, an inflation fluid is introduced from a proximal end of the corresponding catheter to the secondary balloon to inflate the secondary balloon. As a result, the secondary balloon slightly expands the artificial valve outwardly, thereby bringing internal environments of the balloon body at proximal and distal ends thereof into communication with each other. After that, the secondary balloon is deflated. Subsequently, the inflation fluid is introduced from a proximal end of the corresponding catheter to the balloon body to inflate the balloon body until the balloon body is uniformly inflated to its maximum size. This enables stable release of the artificial valve.

In summary, in the delivery catheter, implant delivery system and method of the present invention, through arranging the proximal support member and the distal structure, the risk of sudden axial movement of an implant with respect to the delivery system during delivery and release can be avoided. Moreover, microstructures can be etched in the expandable component to enable the implant to be slightly expanded to bring the internal environments of the balloon body at the proximal and distal ends thereof into communication. This greatly reduces the risk of dislodgement of the implant due to non-simultaneous inflation. Further, the etched portions of the expandable component (e.g., the inflatable balloon or the secondary balloon) can be torn under a high pressure, enabling an inflation fluid to simultaneously enter the internal environments of the balloon body respectively at the proximal and distal ends thereof at the same rate and the same pressure. In this way, the implant can be expanded uniformly in a stable manner, solving the problem of incomplete or asymmetric expansion of the valve during release.

It is to be noted that, as used herein, the terms "first" and "second" are only meant to distinguish various components, elements, steps, etc. from each other rather than indicate logical or sequential orderings thereof, unless otherwise indicated or specified.

It is to be understood that while the invention has been described above with reference to preferred embodiments thereof, it is not limited to these embodiments. In light of the above teachings, any person familiar with the art may make many possible modifications and variations to the disclosed embodiments or adapt them into equivalent embodiments, without departing from the scope of the invention. Accordingly, it is intended that any and all simple variations, equivalent changes and modifications made to the foregoing embodiments based on the substantive disclosure of the invention without departing from the scope thereof fall within this scope.

## Claims

1. A delivery catheter, comprising a catheter, a balloon structure and a guidewire tube, wherein: the balloon structure is disposed at a distal end of the catheter; and the guidewire tube is inserted within the catheter and the balloon structure,
wherein the balloon structure comprises an expandable component and a balloon body, wherein the expandable component is disposed within the balloon body so as to be expandable therein, the expandable component sleeved over a distal end of the guidewire tube, wherein an implant is sleeved over the balloon body and is axially restrained over a middle portion of the expandable component during delivery, wherein the expandable component is configured to provide a radially outward support force from a middle portion of the balloon body during an expansion so as to bring an internal environments of the balloon body at a distal end and a proximal end thereof into communication.

2. The delivery catheter of claim 1, wherein the implant comprises an artificial valve.

3. The delivery catheter of claim 2, wherein the balloon structure further comprises a proximal support member and a distal support member, which are both provided within the balloon body, wherein each of the proximal and the distal support members has a same axial direction as the expandable component, wherein: the proximal support member is sleeved over a proximal end of the expandable component; and the distal support member is sleeved over a distal end of the expandable component, and wherein the artificial valve is axially restrained between the proximal and distal support members during delivery.

4. The delivery catheter of claim 3, wherein the expandable component is an inflatable balloon body comprising a first section, a second section and a third section, that are sequentially connected from a proximal end to a distal end thereof, wherein: the first section is inserted within the proximal support member and is proximally connected to an inner wall of the catheter; the third section is inserted within the distal support member and is distally connected to an outer wall of the guidewire tube; and the second section is located between the proximal and distal support members, and wherein the artificial valve is arranged over the second section during delivery.

5. The delivery catheter of claim 4, wherein each of the first section and the third section has at least one indentation stripe arranged circumferentially, wherein the at least one indentation stripe is provided on an outer wall of the inflatable balloon body, and wherein an extension direction of the indentation stripe is as same as an axial direction of the inflatable balloon body.

6. The delivery catheter of claim 5, wherein the indentation stripe comprises at least one indentation spaced axially.

7. The delivery catheter of claim 6, wherein the expandable component is a secondary balloon, wherein a distal end of the secondary balloon is inserted within the distal support member and is connected to an outer wall of the guidewire tube, and wherein a proximal end of the secondary balloon is inserted within the proximal support member and communicates with the catheter.

8. The delivery catheter of claim 6, wherein the proximal support member is a tubular structure open at both ends, a tapered resilient member or a multi-ridge clamp, and wherein the distal support member is the tubular structure, the resilient member or the multi-ridge clamp.

9. The delivery catheter of claim 8, wherein:
in case of the proximal support member and/or the distal support member being tubular structure(s), a plurality of through-pores are even distributed on an outer circumferential surface of the tubular structure(s); and
in case of the proximal support member and/or the distal support member being tapered resilient member(s), the tapered resilient member(s) comprises a maximum diameter at an end thereof proximal to the artificial valve.

10. An implant delivery system, comprising the delivery catheter of any one of claims 1 to 9.

11. A method for operating an implant delivery system comprising the delivery catheter of any one of claims 4 to 6, comprising:
crimping the implant onto the balloon structure, wherein the balloon body fits against the inflatable balloon, and wherein the implant is axially restrained between the proximal and distal support members;
delivering the implant to a predetermined site;
introducing an inflation fluid from a proximal end of the catheter, wherein the inflation fluid then flows through the catheter into the inflatable balloon, wherein the implant initially expands along an radial direction through the inflatable balloon, and wherein the communication between the internal environments of the balloon body at the distal and proximal ends is established;
increasingly pressurizing the inflation fluid to cause tearing of the indentation stripes under an action of the pressure, bringing the interior of the inflatable balloon into communication with the exterior thereof, wherein the inflation fluid flows at a same rate and a same pressure into the balloon body through the first and third sections;
inflating the balloon body to a maximum size so as to fully expand the implant; and
releasing the implant to the predetermined site.

12. A method for operating an implant delivery system comprising the delivery catheter of claim 7, comprising:
crimping the implant onto the balloon structure, wherein the balloon body fits against the secondary balloon, and wherein implant is axially restrained between the proximal and distal support members;
delivering the implant to a predetermined site;
introducing an inflation fluid from a proximal end of the catheter, wherein the inflation fluid then flows through the catheter into the secondary balloon, wherein the implant initially expands along a radial direction by the secondary balloon, and wherein the communication between the internal environments of the balloon body at the distal and proximal ends is established;
deflating the secondary balloon;
introducing the inflation fluid through the catheter into the balloon body, and inflating the balloon body to a maximum size so as to fully expand the implant; and
releasing the implant to the predetermined site.
